# EUROPEAN PATENT APPLICATION

(11) **EP 4 322 709 A1**
(43) Date of publication of application: **14.02.2024**
(21) Application number: 22784318.2
(22) Date of filing: 01.02.2022
(51) Int. Cl.: H05B 41/36, A61L 9/20, B01J 19/12, H01J 65/00

(54) **ULTRAVIOLET LIGHT RADIATING DEVICE, AND METHOD FOR USING ULTRAVIOLET LIGHT RADIATING DEVICE**

(30) Priority: 07.04.2021 JP 2021065424
(71) Applicant: Ushio Denki Kabushiki Kaisha, Tokyo 100-8150 (JP)
(72) Inventor: YAGYU, Hideaki, Tokyo 100-8150 (JP)
(74) Representative: Maiwald GmbH
(86) International application number: PCT/JP2022/003851
(87) International publication number: WO 2022/215333

(57) **Abstract**

Provided is an ultraviolet light radiating device that can be operated after a short time even if placed in an environment in which stop time continues over an extended period of time. The ultraviolet light radiating device includes: an excimer lamp that emits ultraviolet light; a lighting circuit that applies a lighting voltage to the excimer lamp; and a controller that performs energization control for the lighting circuit. The controller performs control to energize the lighting circuit for a first predetermined time at a predetermined timing.

## Description

### TECHNICAL FIELD

The present invention relates to an ultraviolet light radiating device and particularly relates to an ultraviolet light radiating device including an excimer lamp. The present invention also relates to a method for using the ultraviolet light radiating device.

### BACKGROUND ART

Conventionally, an excimer lamp needs a high voltage when starting lighting after being stopped for a long period of time, and thus has a problem of an increase in the size of a power source for lighting. In view of this, Patent Document 1 below proposes a light irradiation device provided with a UV-LED that radiates ultraviolet light of 390 nm as a start assist light source and applies light emitted from the UV-LED to an excimer lamp to assist the startup.

### PRIOR ART DOCUMENT

### PATENT DOCUMENT

Patent Document 1: JP-A-2017-68944

### SUMMARY OF THE INVENTION

### PROBLEMS TO BE SOLVED BY THE INVENTION

Bacteria (including microbes and fungi) or viruses present in spaces or on object surfaces may cause infectious diseases in humans or animals other than humans, and there is concern that the spread of such infectious diseases may threaten people's lives. Particularly, infectious diseases are likely to spread in facilities where people frequently gather, such as medical facilities, schools, and government offices, and vehicles such as cars, trains, buses, airplanes, and ships, and therefore effective means for inactivating bacteria or viruses are needed. The present applicant has developed an ultraviolet light radiating device equipped with an excimer lamp as a device for performing such an inactivation treatment.

In a case where such an ultraviolet light radiating device is installed in the facilities or the vehicles described above for the inactivation treatment, it is assumed that the unlit state of the excimer lamp continues for a long period of time during, for example, a long vacation in which a business entity that owns or manages the ultraviolet light radiating device is closed for a long period of time. In this case, there is a concern that the excimer lamps will not turn on easily when the ultraviolet light radiating device is intended to be operated again to perform the inactivation treatment.

Not only for inactivation treatment but also for other applications, the continued unlit state of the excimer lamp may make it difficult to operate the lamp afterward.

Given the above problems, an object of the present invention is to provide an ultraviolet light radiating device that can be operated after a short time even if placed in an environment in which stop time continues over an extended period of time.

### MEANS FOR SOLVING THE PROBLEMS

An ultraviolet light radiating device according to the present invention includes an excimer lamp that emits ultraviolet light; a lighting circuit that applies a lighting voltage to the excimer lamp; and a controller that performs energization control for the lighting circuit, the controller performing control to energize the lighting circuit for a first predetermined time at predetermined timing.

According to the ultraviolet light radiating device described above, even if the stopped state continues (the device is stopped), the controller energizes the lighting circuit at a predetermined timing, whereby the excimer lamp is temporarily turned on. Due to lighting, a large amount of electrons are generated in a tube of the excimer lamp. As a result, the startup performance of the excimer lamp is improved because electrons remain in the tube of the excimer lamp at the time the ultraviolet light radiating device is operated next.

The first predetermined time is preferably within five minutes, more preferably within three minutes, and most preferably within one minute. When the lighting operation occurs, electrons are generated in the tube of the excimer lamp, and the generation amount of electrons is not greatly affected by the duration of the lighting operation. Unlike the original purpose of operating the ultraviolet light radiating device, the controller performs the control to energize the lighting circuit at the above-mentioned timing to improve the startup performance when the ultraviolet light radiating device is operated next during the period in which the ultraviolet light radiating device is stopped. Therefore, from the viewpoint of suppressing power consumption and improving the lifetime of the excimer lamp, the first predetermined time is preferably set to be short.

There are several methods for setting the timing at which the controller performs the energization control. As an example, the controller may detect the arrival of the timing at a specific time. As another example, the controller may detect the arrival of the timing at a point at which a second predetermined time has elapsed since the end of the energized state of the lighting circuit.

Regarding the former method, the controller may include, for example, a clock unit that detects a current time, and a storage unit that records information, and information regarding a specific time at which the lighting circuit is energized may be recorded in the storage unit. In addition, regarding the latter method, the controller may include, for example, an elapsed time measurement unit (timer circuit) for measuring the elapsed time, and a storage unit for recording information, and information regarding the elapsed time (first predetermined time) for determining the timing to energize the lighting circuit may be recorded in the storage unit.

In a case where the lighting circuit is already in an energized state when the timing arrives, the controller may perform control to continue the energized state even after the first predetermined time has elapsed.

It is conceivable that, when the controller detects the arrival of the timing at a specific time, the ultraviolet light radiating device is in operation at the specific time. In this case, from the viewpoint of continuing the operation of the ultraviolet light radiating device, the energization to the lighting circuit is continued even after the first predetermined time has elapsed since the arrival of the timing.

In addition, in a case where the controller detects the arrival of the timing at a point at which a second predetermined time has elapsed since the end of the energized state of the lighting circuit, the ultraviolet light radiating device may happen to operate at substantially the same time as the time after the lapse of the second predetermined time. In this case as well, from the viewpoint of continuing the operation of the ultraviolet light radiating device, the energization to the lighting circuit is continued even after the first predetermined time has elapsed since the arrival of the timing.

The second predetermined time is preferably set as a time within 48 hours, and more preferably set as a time within 24 hours. In a case where the ultraviolet light radiating device is not operated for three days or more and is kept on standby without lighting, more than a few seconds are required to again turn on the excimer lamp, and in some cases, the excimer lamp will not light.

When the ultraviolet light radiating device is used for inactivation, a use mode of radiating ultraviolet light toward a space where a person is present is assumed. At the filing date of this application, the threshold limit value (TLV) of an ultraviolet radiation dose per day (eight hours) for a human body is specified depending on wavelength by the American Conference of Governmental Industrial Hygienists (ACGIH) or JIS Z 8812 (Measuring methods of eye-hazardous ultraviolet radiation). From the viewpoint of complying with this standard, it is conceivable to execute an operation mode in which the excimer lamp is intermittently lit when the ultraviolet light radiating device is in operation. Specifically, it is supposed that an operation mode, in which the control of turning on the excimer lamp for about several ten seconds and then turning off the excimer lamp for about several ten seconds is repeated, is executed.

In a case where, for example, the ultraviolet light radiating device is installed in a washroom or the like for inactivation, it may be activated by detecting the presence of a person and may perform the above intermittent operation. In this case, if a delay of about five seconds occurs upon starting the excimer lamp when the ultraviolet light radiating device is operated after being stopped for a long period of time, the time to irradiate a person who first enters the washroom with ultraviolet light for inactivation is short, so that he/she may have insufficient sterilization and inactivation of fingers and the like.

In addition, in the ultraviolet light radiating device that is set to perform the intermittent operation described above during operation, when the time (startup delay time) required to start lighting of the excimer lamp upon activation after the ultraviolet light radiating device has been stopped for a long time is equal to or longer than a lighting period set by the intermittent operation, there is a possibility that the excimer lamp is never turned on during operation.

A time (second elapsed time) from the last turn-off of the excimer lamp mounted on the ultraviolet light radiating device to when the lighting control is performed by the controller is preferably set to a value that can allow the startup delay time at the time of restarting the ultraviolet light radiating device.

The excimer lamp may include a tube filled with a light-emitting gas containing krypton (Kr) and chlorine (CI) or bromine (Br) and a pair of electrodes disposed in contact with an outer surface of the tube at positions spaced apart from each other in a tube axis direction of the tube.

The excimer lamp (hereinafter referred to as "KrCI excimer lamp") including a tube filled with a light-emitting gas containing Kr and Cl generates ultraviolet light having a peak wavelength in the vicinity of 222 nm. The excimer lamp (hereinafter referred to as "KrBr excimer lamp") including a tube filled with a light-emitting gas containing Kr and Br generates ultraviolet light having a peak wavelength in the vicinity of 207 nm.

A low-pressure mercury lamp conventionally used for sterilization exhibits a high emission spectrum about 254 nm close to 260 nm which is a peak wavelength of an absorption spectrum of DNA. It is known that ultraviolet light in such a wavelength band has a risk of affecting a human body when radiated to a human. The skin is divided into three parts from superficial to deep: the epidermis, the dermis, and the hypodermis deeper than the dermis, and the epidermis is further divided into four layers from superficial to deep: the stratum corneum, the stratum granulosum, the stratum spinosum, and the stratum basale. When a human body is exposed to ultraviolet light with a wavelength of 254 nm, the ultraviolet light passes through the stratum corneum, reaches the stratum granulosum or the stratum spinosum or, in some cases, reaches the stratum basale, and is absorbed by DNA of cells present in these layers. This, as a result, causes a risk of skin cancer. Therefore, it is difficult to actively use ultraviolet light in such a wavelength band in places where a human may be present.

On the other hand, a KrCl excimer lamp or a KrBr excimer lamp has a main emission wavelength of less than 240 nm, and thus, has less effect on the human body. Therefore, the ultraviolet light radiating device equipped with such a lamp can be used for the purpose of inactivating bacteria and viruses even in an environment where a human is present.

However, the excimer lamp described above includes a halogen gas such as CI or Br as a light-emitting gas. Halogen has high electronegativity and thus has high electron attachment properties. For this reason, it is considered that, as a result of attaching electrons necessary for discharge by halogen, the above-mentioned excimer lamp is more difficult to start discharge than a lamp filled with another light-emitting gas. Further, in an excimer lamp including an electrode that is disposed on the outer surface of a tube as in the above configuration, electrons are not injected into the tube from the electrode during energization. From this viewpoint, the problem of a startup delay is likely to appear significantly in a KrCI excimer lamp or a KrBr excimer lamp including an electrode that is disposed on the outer surface of a tube, when the lamp is turned on after the unlit state is continued for a long time.

On the other hand, according to the above configuration, even if the operation is stopped for a long time in an ultraviolet light radiating device equipped with the excimer lamp described above, which is prone to startup delay, the lighting circuit is energized by the controller at the predetermined timing to perform a lighting operation, thus creating an environment where electrons are present in the tube. This suppresses the startup delay of the excimer lamp.

Ultraviolet light emitted from the ultraviolet light radiating device equipped with the KrCI excimer lamp or the KrBr excimer lamp can provide sterilization and virus inactivation performance intrinsic to ultraviolet light without causing erythema or keratitis on the skin or eyes of a human or an animal. In particular, taking advantage of a characteristic of being able to be used in an environment where a human is present in contrast to conventional ultraviolet light sources such as low-pressure mercury lamps, the ultraviolet light radiating device can be installed in an indoor or outdoor environment where a human is present to irradiate the entire environment and provide virus inhibition and bacteria elimination in the air and on surfaces of members installed in the environment. This accords with Goal 3 "Ensure healthy lives and promote well-being for all at all ages" included in the Sustainable Development Goals (SDGs) led by the United Nations, and will greatly contribute to the goal target 3.3 "By 2030, end the epidemics of AIDS, tuberculosis, malaria, and neglected tropical diseases and combat hepatitis, water-borne diseases, and other communicable diseases".

The present invention provides a method for using an ultraviolet light radiating device including an excimer lamp that emits ultraviolet light and a lighting circuit that applies a lighting voltage to the excimer lamp, the method including: a step (a) of executing a lighting mode of continuously energizing the lighting circuit to maintain a lighting state of the excimer lamp; and a step (b) of executing a standby mode of stopping energization to the lighting circuit and waiting for transition to the lighting mode, wherein the step (b) includes a step (c) of energizing the lighting circuit for a time within five minutes to perform auxiliary lighting when a predetermined time elapses after the transition from the lighting mode to the standby mode.

According to this method, even in a situation where the ultraviolet light radiating device is not operated for a long time, the excimer lamp can be immediately turned on at the time of operating the ultraviolet radiating device again.

In addition, the present invention provides a method for using an ultraviolet light radiating device including an excimer lamp that emits ultraviolet light and a lighting circuit that applies a lighting voltage to the excimer lamp, the method including: a step (a) of executing a lighting mode of continuously energizing the lighting circuit to maintain a lighting state of the excimer lamp; and a step (b) of executing a standby mode of stopping energization to the lighting circuit and waiting for transition to the lighting mode, wherein the step (b) includes a step (c) of energizing the lighting circuit for a time within five minutes to perform auxiliary lighting when a specific time has come.

According to this method, even in a situation where the ultraviolet light radiating device is not operated for a long time, the excimer lamp can be immediately turned on at the time of operating the ultraviolet radiating device again.

### EFFECT OF THE INVENTION

According to the present invention, the excimer lamp can be turned on after a short time even if placed in an environment in which stop time continues over an extended period of time and then intended to be operated, and the problem of a startup delay is reduced.

### BRIEF DESCRIPTION OF THE DRAWINGS

Fig. 1 is a diagram schematically illustrating a scene in which an ultraviolet light radiating device according to an embodiment of the present invention is used for the purpose of inactivating bacteria or viruses in a room.
Fig. 2 is a schematic diagram illustrating an example of the configuration of the ultraviolet light radiating device.
Fig. 3 is a functional block diagram for describing the configuration of the ultraviolet light radiating device.
Fig. 4A is a perspective view schematically illustrating an example of an external appearance of a light source included in the ultraviolet light radiating device.
Fig. 4B is a perspective view schematically illustrating an example of the external appearance of the light source illustrated in Fig. 4A from which some elements are removed.
Fig. 4C is a cross-sectional view schematically illustrating a positional relationship between a light-emitting tube and electrodes included in the light source.
Fig. 5 is a diagram illustrating an example of the spectrum of ultraviolet light emitted from the light source.
Fig. 6 is a circuit block diagram schematically illustrating a configuration of a lighting circuit included in the ultraviolet light radiating device.
Fig. 7 is a timing chart schematically illustrating the detail of control of the ultraviolet light radiating device.
Fig. 8 is a graph illustrating a relationship between a continuous unlit period and a start time for turning on the light source again.
Fig. 9 is a timing chart schematically illustrating a mode of lighting in a case where the continuous unlit period continues for a long time in a conventional ultraviolet light radiating device.
Fig. 10 is a circuit block diagram schematically illustrating a configuration of a lighting circuit included in the ultraviolet light radiating device.
Fig. 11 is another timing chart schematically illustrating the detail of control of the ultraviolet light radiating device.
Fig. 12 is another timing chart schematically illustrating the detail of control of the ultraviolet light radiating device.

### MODE FOR CARRYING OUT THE INVENTION

An embodiment of the ultraviolet light radiating device and a method for using the same according to the present invention will be described with reference to the drawings as appropriate.

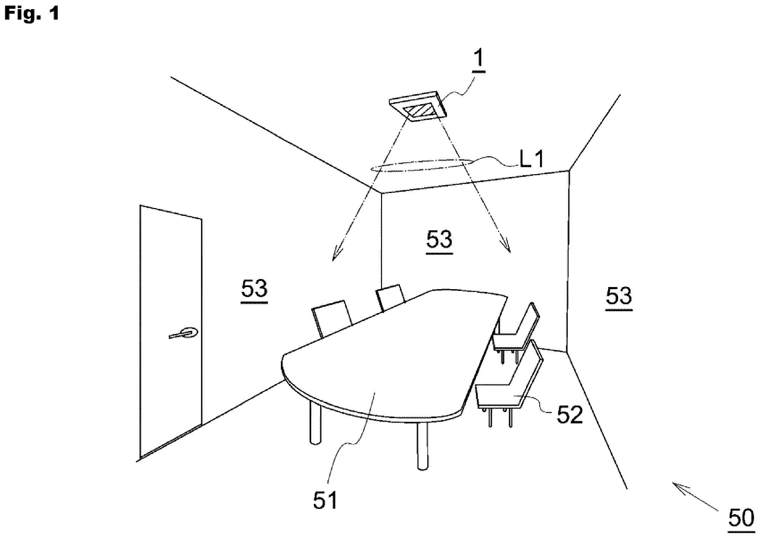

Fig. 1 is a diagram schematically illustrating an example of a scene in which the ultraviolet light radiating device according to the present invention is used to inactivate bacteria or viruses in a room. Fig. 1 illustrates, as an example, a situation in which an ultraviolet light radiating device 1 is installed in a room 50 such as a meeting room. The ultraviolet light radiating device 1 according to the present embodiment is installed to inactivate bacteria on a desk 51, chairs 52, and wallpaper 53 provided in the room 50 and in a space in the room 50.

The ultraviolet light radiating device 1 is configured to emit ultraviolet light L1 which will be described later and to perform an inactivation treatment by applying the ultraviolet light L1 to objects or spaces to be subjected to the inactivation of bacteria.

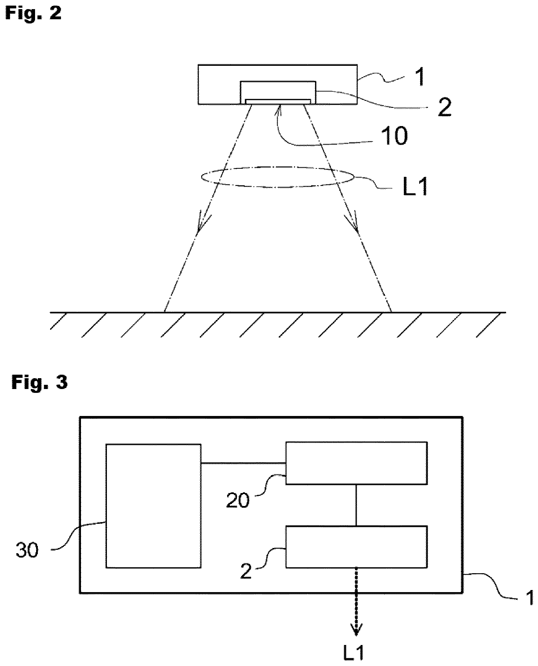

Fig. 2 is a schematic diagram illustrating the configuration of the ultraviolet light radiating device 1. Fig. 3 is a functional block diagram for describing the configuration of the ultraviolet light radiating device 1. The ultraviolet light radiating device 1 includes a light source 2 that emits the ultraviolet light L1, a lighting circuit 20 for lighting the light source 2, and a controller 30 that controls the lighting circuit 20. The lighting circuit 20 and the controller 30 will be described later.

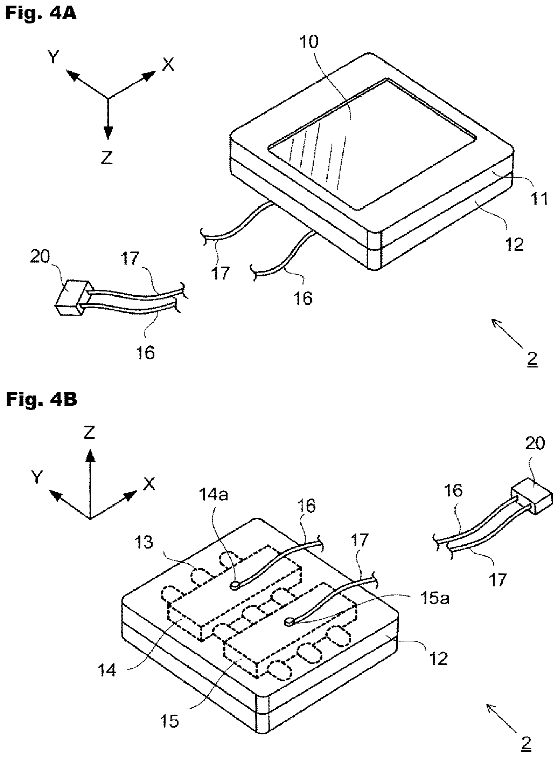

Figs. 4A and 4B are schematic perspective views illustrating the external appearance of the light source 2. It is to be noted, however, that the structure illustrated in Figs. 4A and 4B is merely an example, and the light source 2 of the ultraviolet light radiating device 1 according to the present invention can have any structure.

As illustrated in Fig. 4A, the light source 2 includes a cover 11 having a light extraction surface 10 formed in one of surfaces thereof and a main body casing 12. Fig. 4B is a schematic view of the light source 2 illustrated in Fig. 4A in which a part of the cover 11 is not illustrated. In the example illustrated in Fig. 4B, the light source 2 includes a plurality of light-emitting tubes 13 and electrodes (14, 15) for applying a voltage to the light-emitting tubes 13. The electrodes (14, 15) are connected to power wires (16, 17) via junctions (14a, 15a), respectively. The power wires (16, 17) are connected to the lighting circuit 20.

Fig. 4B illustrates a case where the light source 2 includes three light-emitting tubes 13. However, in the present invention, the number of light-emitting tubes 13 is arbitrary and can be one, two, four, or more.

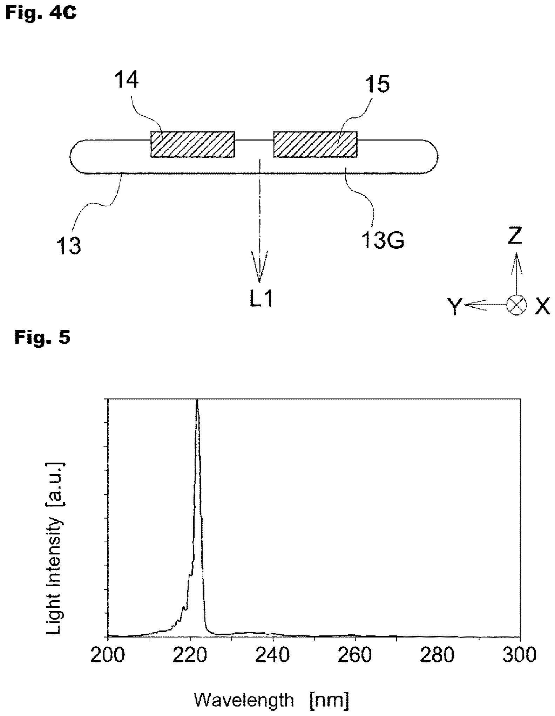

Fig. 4C is a cross-sectional view schematically illustrating a positional relationship between the light-emitting tube 13 and the electrodes (14, 15). As illustrated in Fig. 4C, the electrodes (14, 15) included in the light source 2 are in contact with the outer surfaces of the light-emitting tubes 13, and are disposed at positions separated from each other in the tube axis direction (Y direction). The electrodes (14,15) are made of a conductive material, preferably a material exhibiting reflectivity to the ultraviolet light L1 emitted from the light-emitting tubes 13. For example, the electrodes (14, 15) are both made of Al, an Al alloy, or stainless steel. In the light source 2 according to the present embodiment, the electrodes (14, 15) are disposed so as to extend across the plurality of light-emitting tubes 13 as illustrated in Fig. 4B.

The light-emitting tubes 13 are made of a dielectric material such as quartz glass and are filled with a predetermined light-emitting gas 13G. When a high-frequency voltage of, for example, about 1 kHz to 5 MHz is applied to the electrodes (14, 15), the high-frequency voltage is applied to the light-emitting gas 13G via the light-emitting tubes 13. This generates a discharge plasma inside the discharge space filled with the light-emitting gas 13G and excited atoms of the light-emitting gas form excimers which emit light when transiting to the ground state. The light from the excimers is emitted from the light extraction surface 10 (see Figs. 2 and 4A) as the ultraviolet light L1. That is, in the present embodiment, the light source 2 includes an excimer lamp.

The wavelength of the ultraviolet light L1 emitted from the light source 2 is determined depending on a substance contained in the light-emitting gas 13G filled in the light-emitting tubes 13. For example, when the light-emitting gas 13G contains KrCl, the ultraviolet light L1 emitted from the light source 2 shows a spectrum having a main peak wavelength of about 222 nm. When the light-emitting gas contains KrBr, the ultraviolet light L1 shows a spectrum having a main peak wavelength of about 207 nm. It is to be noted, however, that in the present invention, the type of gas constituting the light-emitting gas 13G to be filled in the light-emitting tubes 13 is not particularly limited and may be appropriately selected depending on a desired wavelength of the ultraviolet light L1. Further, to shift the wavelength to a longer wavelength side, a fluorescent material may be applied onto the tube walls of the light-emitting tubes 13 or the light extraction surface 10.

The main peak wavelength of the ultraviolet light L1 emitted from the light source 2 containing KrCI as the light-emitting gas 13G is described as being "about 222 nm", which is intended to include a difference among individual excimer lamp products and permit not only absolutely precise 222.0 nm but also a wavelength error of ±3.0 nm inclusive from the reference point, 222.0 nm. The same applies to a case where KrBr is contained as the light-emitting gas 13G.

In a case where the ultraviolet light radiating device 1 is assumed to be used for the purpose of sterilization or inactivation in a space in which a human is present, such as the room 50, as schematically illustrated in Fig. 1, the light-emitting gas 13G is preferably selected so that the main emission wavelength is less than 240 nm from the viewpoint of suppressing the influence on the human body. Specifically, the light-emitting gas 13G preferably contains KrCI or KrBr as described above. The term "main emission wavelength" as used herein refers to a wavelength showing a light intensity of 50% or more with respect to a light intensity at a main peak wavelength. Fig. 5 is an example of a spectrum of the ultraviolet light L1 emitted from the light source 2 in a case where the light-emitting gas 13G contains KrCl. In the spectrum in Fig. 5, the main peak wavelength is about 222 nm, and the main emission wavelength is less than 240 nm.

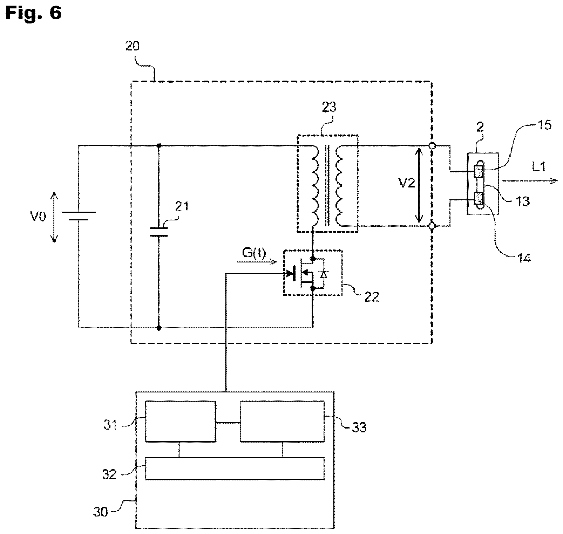

Fig. 6 is a circuit block diagram schematically illustrating the configuration of the lighting circuit 20 included in the ultraviolet light radiating device 1. Fig. 6 also schematically illustrates the controller 30 for controlling the lighting circuit 20 together with the lighting circuit 20.

The lighting circuit 20 is a circuit that converts a DC voltage V0 into a lighting voltage V2 for lighting the light source 2. The DC voltage V0 may be, for example, an output voltage after a commercial AC voltage is converted by an AC/DC converter, or may be an output voltage of a battery (not illustrated).

The lighting circuit 20 illustrated in Fig. 6 is of a type commonly referred to as a flyback type and includes a smoothing capacitor 21, a switching element 22, and a transformer 23. However, in the present embodiment, the lighting circuit 20 of the light source 2 is not limited to the flyback type as long as the lighting circuit 20 can control the energization of the light source 2 based on a control signal from the controller 30.

A primary winding of the transformer 23 is connected to the DC voltage V0 through the switching element 22. The ON/OFF control of the switching element 22 is performed based on a control signal G(t) from the controller 30. When the control signal G(t) changes from Low to High, the switching element 22 shifts from an OFF state to an ON state so that the primary current of the transformer 23 increases with time. Then, when the control signal G(t) changes from High to Low, the switching element 22 shifts from the ON state to the OFF state. At this time, a back electromotive force is generated in a secondary winding of the transformer 23 so that an impulse voltage V2 is generated. When the voltage V2 is applied from the pair of electrodes (14, 15) to the light-emitting tubes 13, the ultraviolet light L1 is emitted from the light source 2. Thereafter, the ON/OFF control of the switching element 22 is repeated, by which the lighting voltage V2 with a high frequency is continuously applied to the excimer lamp, and thus, the ultraviolet light L1 is continuously emitted.

The ultraviolet light radiating device 1 according to the present embodiment has a function of automatically lighting the light source 2 for a predetermined time (short time) when a predetermined time has elapsed since the last turn-off of the light source 2, even if a turn-on instruction is not given from a user of the ultraviolet light radiating device 1.

In the ultraviolet light radiating device 1 according to the present embodiment, the controller 30 includes a control signal generation unit 31, a storage unit 32, and an elapsed time measurement unit 33 as illustrated in Fig. 6. The control signal generation unit 31 is a circuit that generates the control signal G(t) and outputs the generated control signal G(t) to the switching element 22.

The elapsed time measurement unit 33 is a functional means for measuring the elapsed time from the last turn-off of the light source 2 and includes a known timer circuit. The elapsed time measurement unit 33 measures, for example, the elapsed time after the control signal generation unit 31 stops outputting the control signal G(t) to the switching element 22, thereby measuring the elapsed time from the last turn-off of the light source 2.

The ultraviolet light radiating device 1 according to the present embodiment records information regarding a first predetermined time T1 and a second predetermined time T2 in the storage unit 32. When the elapsed time from the last turn-off of the light source 2 reaches the second predetermined time T2 recorded in the storage unit 32, the control signal generation unit 31 generates the control signal G(t) for turning on the light source 2 and outputs the generated control signal G(t) to the lighting circuit 20. As a result, the light source 2 is automatically turned on. Thereafter, when the first predetermined time T1 recorded in the storage unit 32 has elapsed, the control signal generation unit 31 stops outputting the control signal G(t). As a result, the light source 2 is again shifted to the unlit state.

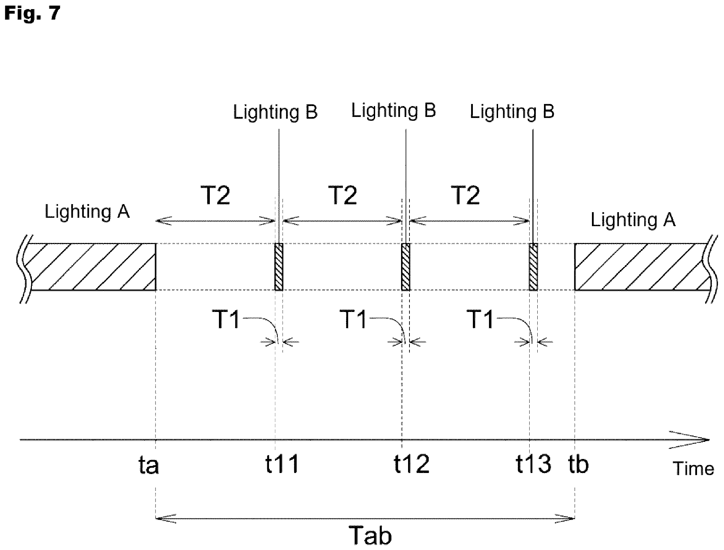

Fig. 7 is a timing chart schematically illustrating a lighting state of the light source 2 included in the ultraviolet light radiating device 1 described above. In Fig. 7, it is assumed that the ultraviolet light radiating device 1 is controlled to the operating state (lighting state of the light source 2) in a time zone before time ta and after time tb according to the turn-on instruction from the user.

When an instruction to start the operation is given from the user to the ultraviolet light radiating device 1 at a certain time (not illustrated) before time ta, the lighting circuit 20 supplies the lighting voltage V2 to the light source 2 so that the light source 2 is turned on and emits the ultraviolet light L1. In the present specification, this state of the ultraviolet light radiating device 1 is referred to as a "lighting mode". For example, when a power start button attached to the ultraviolet light radiating device 1 or provided in a remote controller is operated by the user, the ultraviolet light radiating device 1 executes the lighting mode. The ultraviolet light radiating device 1 maintains the lighting mode until receiving an instruction to stop the operation from the user.

The term "lighting mode" as used herein is not limited to the case of emitting the ultraviolet light L1 continuously at all times (continuous operation mode) from the start of the operation to the stop of the operation, and includes a case of emitting the ultraviolet light L1 intermittently (intermittent operation mode). The intermittent operation mode is an operation mode in which the light source 2 is intermittently lit throughout an operation period, and as a specific example, a mode in which control of lighting the light source 2 for about several ten seconds and then remaining the light source 2 unlit for about several ten seconds is repeated during operation. The lighting period and the unlit period in the intermittent operation mode may be appropriately adjustable in the ultraviolet light radiating device 1, but the unlit period is 2 hours to 2.5 hours at the longest. Further, in the intermittent operation mode, the lighting period and the unlit period may be different.

Note that information regarding the operation mode may also be recorded in the storage unit 32. In addition, the operation mode may be switchable or changeable based on an instruction from the user.

When an instruction to stop the operation is given to the ultraviolet light radiating device 1 from the user at time ta, the supply of the lighting voltage V2 from the lighting circuit 20 to the light source 2 is stopped, so that the light source 2 is turned off. After time ta, the ultraviolet light radiating device 1 continues to stop until the instruction to start the operation is given again by the user. In the present specification, this state of the ultraviolet light radiating device 1 is referred to as a "standby mode".

When the elapsed time from the transition of the ultraviolet light radiating device 1 from the lighting mode to the standby mode reaches the second predetermined time T2 recorded in the storage unit 32, the elapsed time measurement unit 33 outputs a signal indicating this situation to the control signal generation unit 31. When receiving the signal from the elapsed time measurement unit 33, the control signal generation unit 31 outputs the control signal G(t) to the lighting circuit 20 throughout the first predetermined time T1 recorded in the storage unit 32 to turn on the light source 2 even in the standby mode.

In Fig. 7, in order to distinguish lighting of the light source 2 in the lighting mode from in the standby mode, the former is described as "lighting A", and the latter is described as "lighting B". Hereinafter, the same expression ("lighting A", "lighting B") is appropriately used in the present specification.

When the unlit period of the light source 2 including an excimer lamp is continued for a long time, it may take time to activate the light source 2 for turning on again the light source 2, or the light source 2 may not be turned on in some cases. In particular, when the light-emitting gas 13G contains a halogen gas, it tends to take time to turn on the light source 2 again. Furthermore, this phenomenon remarkably appears when the electrodes (14, 15) are formed on the outer surfaces of the light-emitting tubes 13 and no electrode is present in the light-emitting tubes 13 as illustrated in Figs. 4B and 4C.

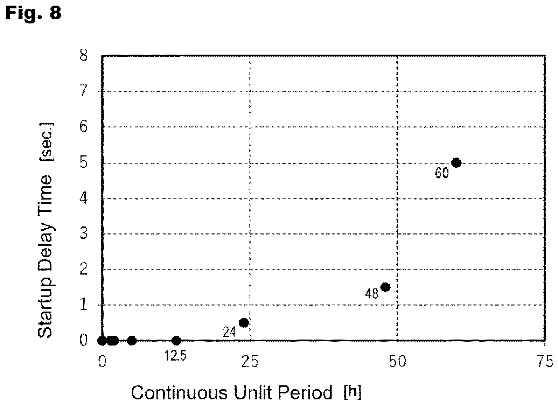

Fig. 8 is a graph illustrating a relationship between the continuous unlit period and the time required for turning on the light source 2 when an instruction to turn on the light source 2 is given in a state where the last continuous unlit period varies. The light source used in the verification was the same structure as the light source 2 illustrated in Figs. 4B and 4C except that there were four light-emitting tubes 13. The light-emitting gas 13G containing krypton (Kr) and chlorine (CI) was enclosed inside the light-emitting tubes 13. Note that, when the light source 2 was turned off, the light source 2 was placed on a placement table with the light extraction surface 10 facing downward.

As illustrated in Fig. 8, when the continuous unlit period is within 24 hours, the time required for startup (startup delay time) is within 0.5 seconds, and when the continuous unlit period is within 48 hours, the startup delay time is within 1.5 seconds. On the other hand, when the continuous unlit period is 60 hours, the startup delay time is 5 seconds. Fig. 8 shows that the startup delay time is likely to increase with an increase in the continuous unlit period, and thus, it is considered that the startup delay time is more than 5 seconds when the continuous unlit period is more than 60 hours. In addition, given the tendency of the graph of Fig. 8, it is considered that the startup delay time is 3 seconds or more when the continuous unlit period exceeds 50 hours.

The reason for the occurrence of the startup delay time is that electrons generated at the time of previous lighting disappear in the light-emitting tubes 13 with time due to an increase in the continuous unlit period, and the number of electrons present in the light-emitting tubes 13 decreases at the time of next lighting. In particular, when the light-emitting gas 13G contains a halogen gas, the halogen gas is likely to attract electrons because of high electronegativity, and electrons necessary for discharge are attached to the halogen gas, so that discharge hardly occurs. Furthermore, in the light source 2 in which the electrodes (14, 15) are provided outside the light-emitting tubes 13 as illustrated in Fig. 4C, electrons cannot be injected into the light-emitting tubes 13 from the electrodes (14, 15), and thus, electrons necessary for discharge are less likely to be generated.

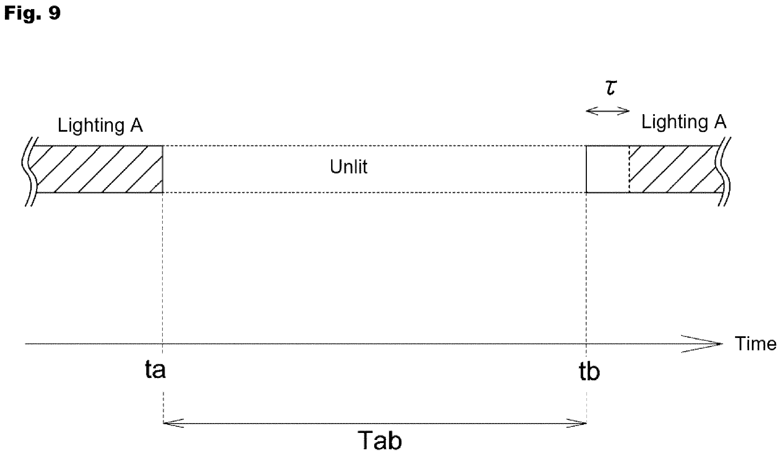

That is, when the continuous unlit period increases, it may take time to start the emission of the ultraviolet light L1 from the ultraviolet light radiating device 1, or in some cases, the light source 2 may not be turned on, if an instruction to start the operation is given from the user to the ultraviolet light radiating device 1. In particular, if the startup delay time is longer than the lighting period set at the time of the intermittent operation in the intermittent operation mode described above set as the lighting mode, the light source 2 may not be turned on even during the lighting mode. For example, as illustrated in Fig. 9, when the period Tab from time ta at which the last turn-off instruction is given to time tb at which the next turn-on instruction is given has a length of several days or longer, the "lighting A" is not performed at time tb but is performed after the startup delay time τ elapses. In some cases, there is a possibility that the unlit state is maintained without the execution of the "lighting A" as described above.

Such a situation may occur, for example, in a case where the ultraviolet light radiating device 1 owned by a business entity such as a company is intended to be operated on a business day after a long vacation.

On the other hand, as illustrated in Fig. 7, the ultraviolet light radiating device 1 according to the present embodiment automatically performs the lighting control even in the standby mode when the second predetermined time T2 elapses from time ta. The second predetermined time T2 is set to a length that can prevent the problem regarding the startup delay time from becoming conspicuous. Accordingly, even if the period Tab from time ta at which the last turn-off instruction is given to time tb at which the next turn-on instruction is given reaches a length of several days or longer, the lighting B is performed once to several times during the period Tab, and thus a state in which electrons remain in the light-emitting tubes 13 is continued. As a result, when the turn-on instruction is issued at time tb, the light source 2 is immediately turned on, and the mode can be shifted to the lighting mode.

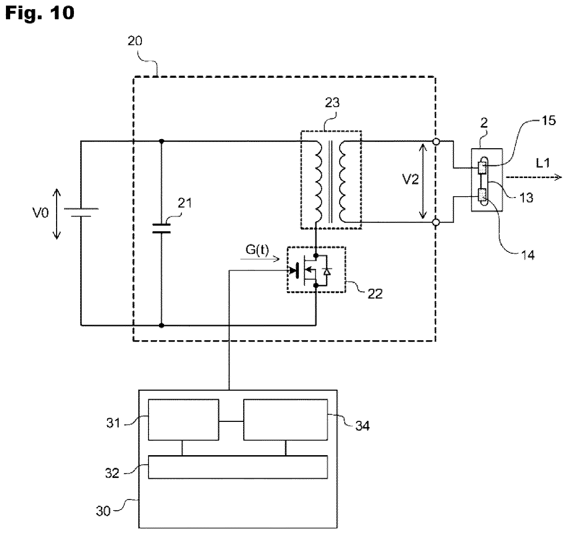

In the above embodiment, the controller 30 performs lighting (lighting B) for a short time based on the elapsed time from the last turn-off even when the ultraviolet light radiating device 1 is in the standby mode. As another configuration, the controller 30 may perform control to execute the lighting B based on the current time. Fig. 10 illustrates the configurations of a controller 30 and a lighting circuit 20 according to the other embodiment in a manner similar to Fig. 6.

In this configuration, the controller 30 includes a control signal generation unit 31, a storage unit 32, and a clock unit 34. The clock unit 34 is a means having a function of detecting the current time. Specifically, the clock unit 34 may be an atomic clock or may be configured by a processor to detect the current time by communicating with another device.

The ultraviolet light radiating device 1 according to this embodiment records information regarding a specific time and a first predetermined time T1 in the storage unit 32. When the current time reaches the specific time recorded in the storage unit 32, the control signal generation unit 31 generates the control signal G(t) for turning on the light source 2 and outputs the generated control signal G(t) to the lighting circuit 20. As a result, the light source 2 is automatically turned on. Thereafter, when the first predetermined time T1 recorded in the storage unit 32 has elapsed, the control signal generation unit 31 stops outputting the control signal G(t). As a result, the light source 2 is again shifted to the unlit state.

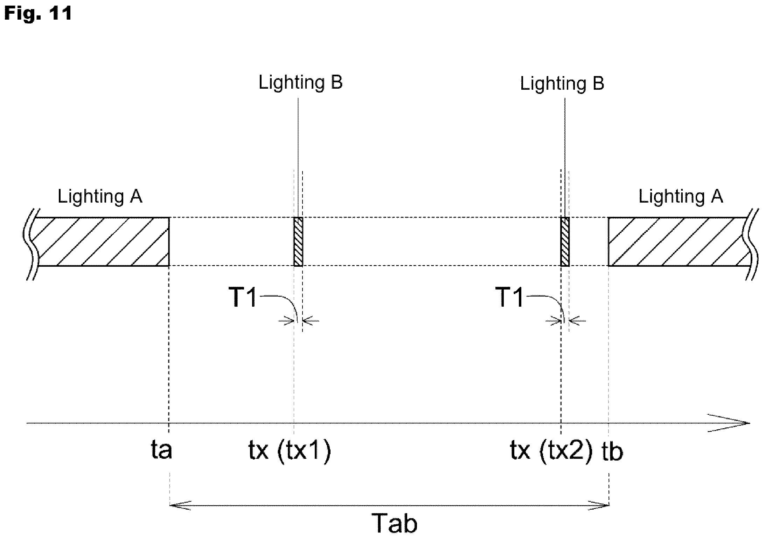

Fig. 11 is a timing chart schematically illustrating the lighting state of the light source 2 included in the ultraviolet light radiating device 1 in a manner similar to Fig. 7 in a case where the lighting B is performed based on time. In Fig. 11, it is assumed that, as in Fig. 7, the ultraviolet light radiating device 1 is controlled to the operating state (lighting state of the light source 2) in a time zone before time ta and after time tb according to the turn-on instruction from the user.

When an instruction to stop the operation is given to the ultraviolet light radiating device 1 from the user at time ta, the supply of the lighting voltage V2 from the lighting circuit 20 to the light source 2 is stopped, so that the light source 2 is turned off. Thus, the ultraviolet light radiating device 1 shifts from the lighting mode to the standby mode.

In the present embodiment, it is assumed that one specific time tx within 24 hours is recorded in the storage unit 32. When detecting that the current time reaches the specific time tx recorded in the storage unit 32, the clock unit 34 outputs a signal indicating this situation to the control signal generation unit 31. When receiving the signal from the elapsed time measurement unit 33, the control signal generation unit 31 outputs the control signal G(t) to the lighting circuit 20 throughout the first predetermined time T1 recorded in the storage unit 32 to turn on the light source 2 (perform the lighting B) even in the standby mode. Thereafter, the light source 2 is turned off.

Further, when the standby mode is continued and the clock unit 34 detects that the current time reaches the specific time tx, the same control as described above is performed and the lighting B is executed. In Fig. 11, the time tx at which the first lighting B is executed and the time to at which the second lighting B is executed are the same in the sense of time expressed in a 24-hour system, but are different in the absolute sense because the dates are different. In order to indicate this respect, in Fig. 11, the time at which the first lighting B is executed is described as to (tx1), and the time at which the second lighting B is executed is described as tx (tx2).

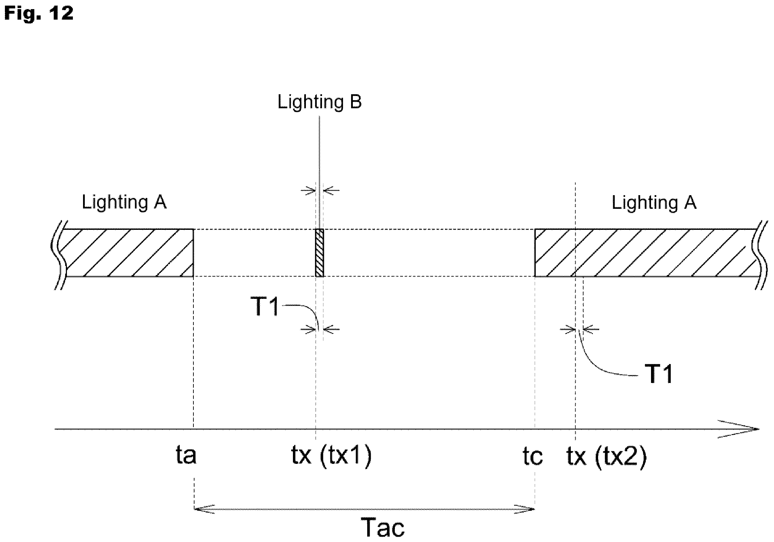

In the control method illustrated in Fig. 11, it is conceivable that the ultraviolet light radiating device 1 is in operation (lighting mode) in response to the instruction from the user at the time point the current time reaches the specific time tx (see Fig. 12). Fig. 12 is a timing chart schematically illustrating the lighting state of the light source 2 included in the ultraviolet light radiating device 1 in a manner similar to Fig. 11 in a case where the lighting B is performed based on time. Fig. 12 illustrates a case where the ultraviolet light radiating device 1 is given the instruction to start the operation from the user at time tc before the time to (tx2), and the light source 2 is currently turned on (lighting A).

In this case, when the clock unit 34 notifies that the current time reaches the specific time tx, the control signal generation unit 31 detects that the lighting mode is currently executed, and generates the control signal G(t) so as to continue the lighting A. That is, unlike the case of Fig. 11, the light source 2 is not turned off even when the first predetermined time T1 elapses from the time to (tx2).

Note that information regarding a specific time including one or more times within 24 hours may be recorded in the storage unit 32. In other words, when the standby mode continues for 24 hours or more, the lighting B may be executed twice or more.

Furthermore, in this embodiment, the storage unit 32 may record information regarding the scheduled time (first specific time) at which the lighting B is scheduled to be executed and the scheduled time (second specific time) at which the light source 2 is to be turned off at the time of execution of the lighting B. In this case, the storage unit 32 does not necessarily need to record the information regarding the first predetermined time T1.

### [Other embodiments]

Other embodiments will now be described.
<1> The light source 2 may include an LED light source (not illustrated) for start assist. In this case, when a condition for executing the lighting B is established, the controller 30 may output the control signal G(t) to the switching element 22 and perform lighting control on the LED light source for start assist. When a condition for stopping the lighting B is generated, more specifically, when the first predetermined time T1 elapses after the lighting B is started, the output of the control signal G(t) to the switching element 22 may be stopped, and the LED light source may be turned off.
<2> The above embodiment has described an example in which the instruction to start/end the lighting mode (lighting A) is given by the user. However, the controller 30 may automatically control the start/end of the lighting mode based on a time schedule recorded in the storage unit 32. In this case, when the time schedule is set in consideration of the business day of the business entity that owns or manages the ultraviolet light radiating device 1, problems such as a startup delay and non-lighting may also occur upon the startup after a long vacation. Thus, due to the execution of the lighting B during the standby mode as in the ultraviolet light radiating device 1 described above, the above-mentioned problem can be solved.
<3> When turning on the light source 2 in the standby mode (upon the start of the lighting B), the controller 30 may control the switching element 22 so that the voltage V2 input to the light source 2 is higher than that in the lighting mode. As a result, even in a case where the standby mode continues for a long period of time, the light source 2 can be reliably turned on during the standby mode to suppress the disappearance of electrons.
<4> The information regarding the second predetermined time T2 or the information regarding the specific time (tx or the like) recorded in the storage unit 32 may be updated according to the long-term use of the ultraviolet light radiating device 1. More specifically, the second predetermined time T2 may be shortened, or the number of specific times within 24 hours may be increased.
<5> In the above embodiment, the lighting B for a short time is automatically performed even when the controller 30 is in the standby mode based on the information regarding the elapsed time (second predetermined time) or the information regarding the specific time recorded in the storage unit 32. However, the lighting B for a short time may be performed based on an instruction from the user.
<6> The above embodiment has described the case where the ultraviolet light radiating device 1 is used for the purpose of inactivating bacteria and viruses in the room 50. However, the present invention is not limited to the application of the ultraviolet light radiating device 1. When the ultraviolet light radiating device 1 includes an excimer lamp that emits the ultraviolet light L1 as the light source 2 and a situation is assumed in which the ultraviolet light radiating device 1 is turned on after a continuous unlit state for a long time, the effect is expected regardless of the application.
<7> The configuration of the light source 2 (excimer lamp) described above with reference to Figs. 4A to 4C is merely an example. As an example, the light source 2 may have a structure in which two light-emitting tubes 13 are provided in a concentric manner, and the light-emitting gas 13G is filled between the inner tube and the outer tube (double tube structure). As another example, the light source 2 may have a structure (single tube structure) in which electrodes are provided inside and outside a single light-emitting tube 13 filled with the light-emitting gas 13G. Furthermore, as still another example, the light source 2 may have a structure (flat tube structure) in which electrodes (14, 15) are provided on two facing surfaces of the light-emitting tube 13 that has rectangular surfaces and that is filled with the light-emitting gas 13G.

### DESCRIPTION OF REFERENCE SIGNS

- 1: Ultraviolet light radiating device
- 2: Light source
- 3: Goal
- 11: Cover
- 12: Main body casing
- 13: Light-emitting tube
- 13G: Light-emitting gas
- 20: Lighting circuit
- 21: Smoothing capacitor
- 22: Switching element
- 23: Transformer
- 30: Controller
- 31: Control signal generation unit
- 32: Storage unit
- 33: Elapsed time measurement unit
- 34: Clock unit
- 50: Room
- 51: Desk
- 52: Chair
- 53: Wallpaper
- L1: Ultraviolet light
- T1: First predetermined time
- T2: Second predetermined time
- tx: Specific time

## Claims

1. An ultraviolet light radiating device comprising:
an excimer lamp that emits ultraviolet light;
a lighting circuit that applies a lighting voltage to the excimer lamp; and
a controller that performs energization control for the lighting circuit,
the controller performing control to energize the lighting circuit for a first predetermined time at a predetermined timing.

2. The ultraviolet light radiating device according to claim 1, wherein, in a case where the lighting circuit is already in an energized state when the timing arrives, the controller performs control to continue the energized state even after a lapse of the first predetermined time.

3. The ultraviolet light radiating device according to claim 1 or 2, wherein the controller detects arrival of the timing at a specific time.

4. The ultraviolet light radiating device according to claim 1 or 2, wherein the controller detects arrival of the timing at a point at which a second predetermined time has elapsed since an end of an energized state of the lighting circuit.

5. The ultraviolet light radiating device according to claim 4, wherein the second predetermined time is within 48 hours.

6. The ultraviolet light radiating device according to claim 1 or 2, wherein the first predetermined time is within five minutes.

7. The ultraviolet light radiating device according to claim 1 or 2, wherein the excimer lamp includes a tube filled with a light-emitting gas containing krypton (Kr) and chlorine (CI), and a pair of electrodes disposed in contact with an outer surface of the tube at positions spaced apart from each other in a tube axis direction of the tube.

8. A method for using an ultraviolet light radiating device including an excimer lamp that emits ultraviolet light and a lighting circuit that applies a lighting voltage to the excimer lamp, the method comprising:
a step (a) of executing a lighting mode of continuously energizing the lighting circuit to maintain a lighting state of the excimer lamp; and
a step (b) of executing a standby mode of stopping energization to the lighting circuit and waiting for transition to the lighting mode,
wherein the step (b) includes a step (c) of energizing the lighting circuit for a time within five minutes to perform auxiliary lighting when a predetermined time elapses after the transition from the lighting mode to the standby mode.

9. A method for using an ultraviolet light radiating device including an excimer lamp that emits ultraviolet light and a lighting circuit that applies a lighting voltage to the excimer lamp, the method comprising:
a step (a) of executing a lighting mode of continuously energizing the lighting circuit to maintain a lighting state of the excimer lamp; and
a step (b) of executing a standby mode of stopping energization to the lighting circuit and waiting for transition to the lighting mode,
wherein the step (b) includes a step (c) of energizing the lighting circuit for a time within five minutes to perform auxiliary lighting when a specific time has come.

10. The method for using an ultraviolet light radiating device according to claim 8 or 9, wherein the excimer lamp includes a tube filled with a light-emitting gas containing krypton (Kr) and chlorine (CI) or bromine (Br), and a pair of electrodes disposed in contact with an outer surface of the tube at positions spaced apart from each other in a tube axis direction of the tube.
